# EUROPEAN PATENT SPECIFICATION

(11) **EP 2 778 548 B1**
(45) Date of publication and mention of the grant of the patent: **23.08.2017**
(21) Application number: 11875557.8
(22) Date of filing: 07.11.2011
(51) Int. Cl.: F24D 13/02, F24H 3/00, A61L 9/16, B03C 3/28, B03C 3/00, F24F 3/14, F24F 3/16, A61L 9/22

(54) **ROOM AIR-CONDITIONING DEVICE**
RAUMKLIMAANLAGE
DISPOSITIF DE CONDITIONNEMENT DE L'ATMOSPHÈRE D'UN LIEU

(43) Date of publication of application: 17.09.2014
(73) Proprietor: Infrarrojos Para el Confort S.L., 41800 Sanlúcar la Mayor (Sevilla) (ES)
(72) Inventor: SÁNCHEZ TÁVORA, Eugenio José, E-41020 Sevilla (ES)
(74) Representative: Pons
(86) International application number: PCT/ES2011/070766
(87) International publication number: WO 2013/068606

(56) References cited:
- EP-A1- 0 958 851
- EP-A1- 2 101 120
- EP-A2- 0 251 783
- WO-A2-2009/061113
- BE-A- 899 045
- CN-A- 102 198 286
- CN-U- 2 123 723
- CN-Y- 201 098 387
- JP-A- 2002 350 060
- KR-A- 20110 049 465
- KR-B1- 100 826 998
- KR-B1- 101 028 946
- US-A1- 2006 130 663

## Description

### OBJECT OF THE INVENTION

The present invention can be included within the technical field of room air conditioning, in particular the conditioning of the moisture and hygiene of the atmosphere of said room.

The object of the present invention relates to a device used for conditioning moisture and the conditions of asepsis of a room.

### BACKGROUND OF THE INVENTION

On numerous occasions, the level of comfort of spaces such as homes, places of work, hospital rooms, hotels, etc., is very reduced, especially due to one of the following two factors:
- a high degree of moisture in walls and/or floors, which causes the room occupants not only to feel an increased sensation of cold but also lung diseases, as well as the deterioration of the goods contained in the room;
- a deficient air renewal in the room, which causes a reduced quality of said air, apparent in the presence of odours which make the wellbeing of the occupants decrease, and produces a high pollution level. It is therefore desired to give the atmosphere of a room improved conditions in terms of health and comfort for the occupants.

International application WO2009061113A2 (LEE SANG JUN), against which claim 1 is delimited, discloses a heating apparatus for thermal insulating in room temperature by a method of manufacturing a temperature-maintaining heating apparatus. The heating apparatus includes an electric heating plate, an electric heating cable, a bimetal, first and second contact terminals, a termperature sensor, heat insulating material, glass wool, a frame, a triangular support plate, and a control unit. The control unit is configured to control the supply of power though a pair of lead wires, and is also configured to receive temperature data, which is detected by the temperature sensor, through the lead wires of the temperature sensor and control radiant heat temperature.

Korean patent KR100826998B1 (GREEN CELL) refers to a heat radiating apparatus for a greenhouse or a cattle shed to allow the heat generated from a heater rod to be effectively transferred to a heat radiating pin by using packing, a plug and a nut, and to obtain antibacterial, antifungal and deodorizing effects by forming an antibacterial/anion-discharging coating layer on a heat radiating pin. A heat radiating apparatus for a greenhouse or a cattle shed comprises: a heat radiating pin which is formed on the circumferential surface of a main body; an antibacterial/anion-discharging coating layer which is formed on the heat radiating pin; a receiving space of which the inner circumferential surface is fit to the circumferential surface of a heater rod; a plug and a packing, both of which seal up one end of the receiving space; a nipple or nut and a packing, both of which seal up the other end of the receiving space; and an electric wire which is drawn outward through the nut and the packing. The document illustrates air cleaning function and deodoration effect by using zeolite, as well as it further discloses an antibacterial effect associated to nano silver mixed with a paint.

European application EP2101120A1 (LIBRIZZI, GIUSEPPE) relates to the field of ducts for conveying air for environmental conditioning, of panels for said ducts and of the relative joining means, with at least one internal face for metallic or plastic material, treated with a solution of silver ions and zeolite having an antimicrobial function.

Korean patent KR101028946B1 (KIM TAE WOONG) relates to a ceramic coating agent for far infrared radiation, which includes, based on 100 parts by weight of the coating agent, 10∼35 parts by weight of filler consisting of SiC, Fe2O3 and MnO, 20∼35 parts by weight of silane, 1∼5 parts by weight of silver(Ag), 0.5∼1 parts by weight of cerium (Ce) or platinum (Pt), and 1∼5 parts by weight of rare earth metals. In the filler, a weight ratio of SiC: Fe2O3: MnO is 7:2:1.

### DESCRIPTION OF THE INVENTION

The present invention overcomes the technical problem posed, by a room air-conditioning device according to claim 1, which comprises a framework which supports and/or contains a first body that emits negative ions, which causes an ionization of the particles present in suspension in the atmosphere of said room, thereby achieving the effects stated below:
- the precipitation of said particles, which has the consequence of the elimination of odours from said particles, as well as cleaning of the atmospheric air, avoiding breathing problems, particularly to occupants with allergy problems to any type of particle, pollen, dust mites, etc..
- a considerable increase in wellbeing of the room's occupants, since the negative ionization of the atmospheric air causes relaxation and stimulates blood pressure regulation.

According to a preferred embodiment, the device further incorporates, preferably contained in and / or supported by the framework, an aseptic compound to eliminate bacteria and microbes from the atmosphere. Preferably, the aseptic compound is nanosilver. Nanosilver is based on silver ions which have biosterilizing capacity, capable of eliminating up to 650 different types of microbes and bacteria in its environment, since nanosilver directly affects the metabolic function of said microbes and bacteria, causing the rupture of its outer membrane.

The device further incorporates, preferably contained in and/or supported by the framework, emission means, in order to emit infrared radiation, to dry out the walls and / or objects contained in the room, thereby removing moisture from the atmosphere and its negative consequences in terms of thermal comfort and respiratory conditions, among others.

The emission means comprises a second body which is adapted in order to emit infrared light by heating, wherein the emission means further comprises a power source to provide energy to heat said second body. The infrared light has a wavelength of approximately 8 micrometres. The power source comprises a source of electricity connected to an electrical conductor wire which runs through the interior of the second body, wherein the second body comprises a first plate with substantially uniform thickness.

Preferably, the second body comprises a heat-conducting material, for example anodized aluminium. Preferably, the second body comprises a sheet, which is preferably coated on at least one of its surfaces with a ceramic coating (preferably paint) that emits negative ions. Preferably, the coating is a paint comprising silver-doped zeolite elements.

As stated above, preferably, the first body is a layer of ceramic paint, having substantially uniform thickness, and distributed on an outer surface of the first plate of the second body. For example, the first plate may be an anodized aluminium plate coated with a ceramic paint based on silver-doped zeolite elements, which constitutes, on the one hand, an emitter of negative ions in accordance with the first body and, on the other hand, a nanosilver compound with biocide and aseptic activity.
Additionally, the second body further comprises a second plate equipped with a winding channel on one of its surfaces to house the conductor wire, wherein the conductor wire is enclosed between the first plate and the second plate.

Preferably, the device of the invention incorporates protection means adapted to cause the disconnection of the supply of the power source to the second body. Preferably, the protection means comprise a thermostat adapted to cut-off the supply of the power source when the wire has reached a temperature greater than a predetermined threshold value.

The source of electricity may be DC or AC, which gives the invention greater versatility, expanding its scope of application to highly varied places, such as domestic or industrial installations, as well as special installations, such as means of transport, particularly prone to problems of damp, odours and bacteria, e.g. boats, aeroplanes, buses, lorries, etc. In the case of DC, solar photovoltaic generators are preferred.

The framework may be made in various materials, depending on the conditions of use. In particular, for corrosive atmospheres, corrosion-resistant plastic materials are preferably used, whilst for not especially corrosive atmospheres, aluminium is the preferred material, due to its reduced weight and great ease of handling. In addition, in the case of corrosive atmospheres, it is foreseen the disposal of an additional cover sealed with a rubber gasket.

The device of the invention may incorporate control means, comprising a controller and at least one temperature measuring device which is located in the room, where the controller indicates the activation and deactivation of the power source depending on the temperature measurement detected by the temperature measuring device.

From that explained above, it is gathered that the present invention allows the air conditioning of a room by purification of the air of said atmosphere, as well as, where applicable, the elimination of bacteria and microbes and, also, where applicable, of the damp.

### DESCRIPTION OF THE DRAWINGS

To complement the description being made and in order to aid towards a better understanding of the characteristics of the invention, in accordance with a preferred example of practical embodiment thereof, a set of drawings is attached as an integral part of said description wherein, with illustrative and non-limiting character, the following has been represented:
Figure 1 - Shows a representative diagram of the structure of the device.
Figure 2 - Shows a perspective schematic view of the heating means.

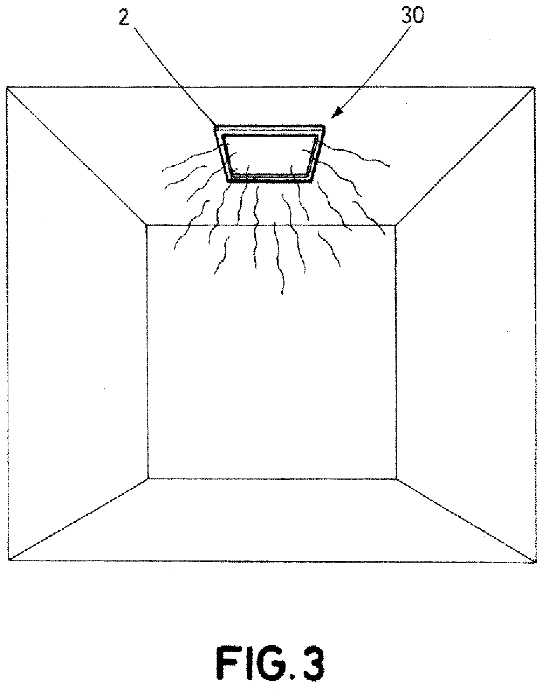

Figure 3 - Shows a diagram of the device installed in a room.

### PREFERRED EMBODIMENT OF THE INVENTION

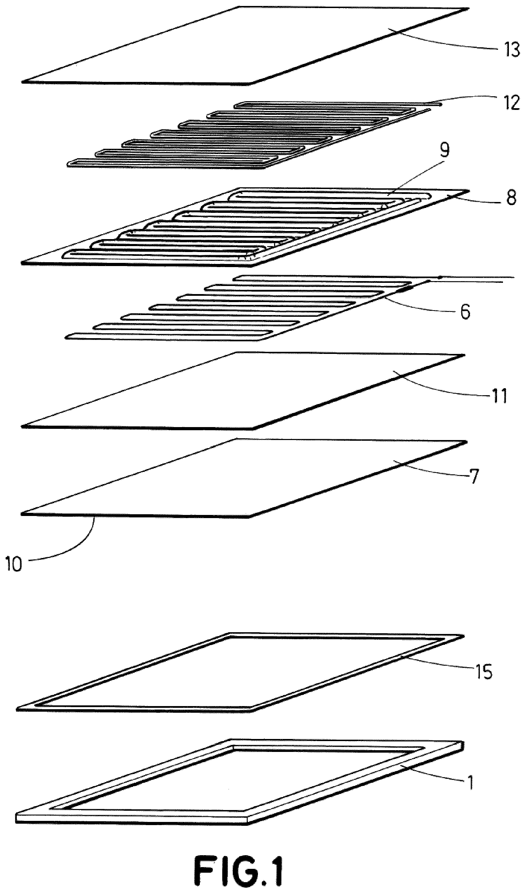

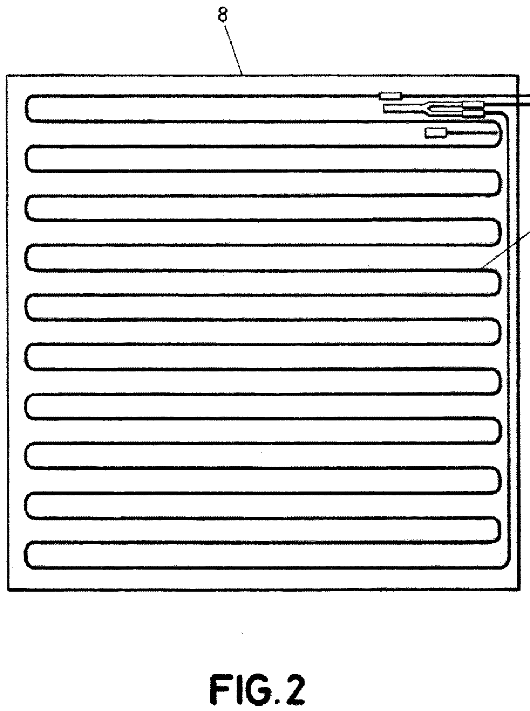

A detailed explanation of a preferred embodiment of the invention is given below with the aid of figures 1 and 2 attached.

The room air-conditioning device (30) according to the present invention comprises a framework (1, 2), which comprises a frame (1) and a counter-frame (2), where the frame (1) serves to hold the elements disposed inside and the counter-frame (2) serves to close and secure said elements. A first aluminium plate (7) and a second aluminium plate (8), both of anodized aluminium are housed in the interior of the framework (1, 2). The first aluminium plate (7) is disposed towards the exterior and on its outer surface a layer of ceramic paint (10) with substantially uniform thickness is distributed, where said ceramic paint (10) incorporates an ionizing compound comprising silver-doped zeolite compounds, which allows passive emission (without energy consumption) of negative ions.

The silver-doped zeolite elements also constitute a nanosilver compound to eliminate microbes and bacteria. The second aluminium plate (8) is disposed on the first aluminium plate (7), towards the interior of the device (30), and is formed to have on its surface a channel (9) according to a winding path to house therein a conductor wire (6) which describes a winding path in the interior of said channel (9).

The conductor wire (6) is connected to a source of electricity (not shown), to heat the first aluminium plate (7) and the second aluminium plate (8) to a substantially uniform temperature, a consequence of which, the layer of ceramic paint (10) emits an infrared radiation, preferably of a wavelength of 8 micrometres. Said infrared emission causes the drying out of walls, floor and possibly other objects disposed in the room, thereby removing moisture. The conductor wire (6) may have a diameter of approximately 1 mm.

The conductor wire (6) passing along the winding channel (9) all throughout the second aluminium plate (8) guarantees great precision in the uniform temperature distribution. The conductor wire (6) is preferably made in FeCrAl alloy, and has an electrical insulation coating (not shown), particularly of ETFE, capable of withstanding temperatures of up to 250 °C.

Between the first aluminium plate (7) and the second aluminium plate (8), there is a first layer of sealing epoxy resin (11) loaded with aluminium particles, applied in a vacuum, to improve the conductivity of the conductor wire (6), which is, in turn, enclosed between the two layers of aluminium (7, 8), avoiding contact with the air, so as to produce through said first layers of epoxy resin (11) a minimization of heat loss on increasing the thermal conductivity between the conductor wire (6) and the first aluminium plate (7), which leads to a greater uniformity in the energy distribution of the conductor wire (6) to said first aluminium plate (7). In addition, there is a second layer of epoxy resin (12) covering the second aluminium plate (8), to guarantee conductivity between both aluminium plates (7, 8).

With the aim of achieving the greatest possible thermal uniformity in the second aluminium plate (8), the conductor wire (6) must be in as close contact as possible within the aluminium, attempting to avoid to the greatest possible extent accumulations of small air sacs which would cause, on the one hand, the lack of temperature uniformity on the radiant surface of the plate, with the consequent decrease in performance and the poorer quality of infrared emission, as well as a shortening of the working life of the conductor wire (6) since there would be points along the conductor wire (6) with different temperature to the rest.

The type of epoxy resin used is a high temperature resin loaded with aluminium which gives high thermal conductivity. The thickness determined for the resin layer is preferably 0.2 mm, to achieve a good adherence and thermal contact not only with the conductor wire (6) but also between aluminium plates (7, 8). A smaller thickness would not guarantee the absence of air between the aluminium plates (7, 8), which would give rise to an undesired loss of conductivity.

There is also an insulator (15) between the ceramic paint (10) and the frame (1), said insulator in the form of tape adapted to the form of the frame.

Depending on the distance from the device (30) at which said walls, ceiling and objects to dry out are located, the first aluminium plate (7) must reach higher or lower temperatures. By way of example, for applications of up to 1.2 m distance, the first aluminium plate (7) may be at a temperature of 60 °C, whilst for greater distances, for example, 5 m, the temperature may be 230 °C.

To avoid heat losses through the rear part of the device, a layer of thermal insulator (13) is disposed, preferably an aerogel (13) due to its extremely low thermal conductivity, in the order of 0.03 W/m*K, leaving the second layer of resin (12) between the second aluminium plate (8) and the aerogel plate (13), to avoid contact between the second aluminium plate (8) and the aerogel plate (13). The layer of aerogel (13) further provides a high degree of impermeability and fire resistance of grade MI.

The device is controlled by a thermostat (not shown) incorporated inside the framework (1, 2), to avoid excessive heating, for example, it stops the operation of the source of electricity when a temperature of 220 °C is reached inside the framework (1, 2).

The electrical connection from the conductor wire (6) to the source of electricity (which may be a battery or similar, or also the mains electricity), is carried out preferably through a cold glue formed by aluminium electric conductor of 1.5 mm² section coated with a high temperature thermal and electrical insulation cover.

The power source may operate both in AC and DC, so that the device can be used both in domestic and industrial applications, and in special applications such as boats, aeroplanes, buses, lorries, etc., which are facilities prone to having problems of damp, as well as odours and bacteria. In the case of DC, at least one solar photovoltaic collector (not shown)is preferred as source of electricity.

The framework (1, 2) is manufactured in materials that depend on the conditions of the environment where the device is going to be used. In particular, for aggressive atmospheres, a corrosion-resistant plastic is used, whilst for non-aggressive atmospheres, the framework (1, 2) may be of aluminium as it is easy to handle and lightweight. Likewise, for the case of aggressive atmospheres, the framework (1, 2) is sealed by a rear cover (not represented) of the same plastic material attached to the framework (1, 2) by flaps and screws and including a rubber sealing gasket (not shown) to protect from damp.

To guarantee the correct operation of the device (30) in the function of drying materials, it incorporates control means (not shown), comprising a controller and at least one temperature measuring device connected to the controller, said temperature measuring device being located in the room, where the controller causes the activation and deactivation of the power source depending on the temperature measured by the temperature measuring device. The controller may be connected to the temperature measuring device in various ways, e.g. electrically or via infrared.

The device (30) may take on a multitude of forms, not only square or rectangular, but it may be developed in the form required depending on the application. Likewise, despite the fact that the colour of the visible side is normal white colour, colouring pigments may be used to give the desired colour in accordance with the location of the device. The surfaces of the framework (1, 2) allow them to be simply fixed to any surface, depending on the location requirements of the device (30).

## Claims

1. Room air-conditioning device comprising:
- a framework (1, 2),
- a first body (10) for emitting negative ions, disposed inside the framework (1, 2), to cause an ionization and precipitation of particles present in suspension in the atmosphere of said room, to purify the air and eliminate bad odour,
- emission means (6, 7, 8, 9) for emitting infrared radiation, to dry out the walls and / or objects contained in the room, the emission means comprising:
- an infrared radiation emitting second body (7, 8, 9) configured so as to emit infrared light by heating, and
- a power source for providing energy to heat the second body (7, 8, 9), the power source comprising a source of electricity connected to the second body (7, 8, 9) through an electrical conductor wire (6),
wherein the second body (7, 8, 9) comprises:
- a first plate (7) having substantially uniform thickness, and **characterised in that** the second body also comprises
- a second plate (8) equipped with a winding channel (9) on one of its surfaces to house the conductor wire (6),
wherein the conductor wire (6) is enclosed between the first plate (7) and the second plate (8).

2. Room air-conditioning device, according to claim 1, wherein the first body (10) is a body capable of passively emitting negative ions, without energy consumption.

3. Room air-conditioning device, according to any of claims 1 and 2, wherein the second body (7, 8, 9) has the form of a plate.

4. Room air-conditioning device, according to any of claims 2 and 3, wherein the first body (10) is a negative ion emitting ceramic coating.

5. Room air-conditioning device, according to claim 4, wherein the ceramic coating is a paint comprising silver-doped zeolite elements.

6. Room air-conditioning device, according to any of claims 1 to 5, wherein the second body (7, 8, 9) is manufactured in a heat-conducting material.

7. Room air-conditioning device, according to claim 6, wherein the material is anodized aluminium.

8. Room air-conditioning device, according to claim 1, wherein it further incorporates an aseptic compound to eliminate bacteria and microbes from the atmosphere.

9. Room air-conditioning device, according to claim 8, wherein the aseptic compound contains nanosilver.

10. Room air-conditioning device, according to claims 5 and 9, wherein the ceramic paint with silver-doped zeolite elements constitutes the nanosilver-based aseptic compound.

11. Room air-conditioning device, according to claim 1 wherein the first aluminium plate (7) incorporates the ceramic paint.

12. Room air-conditioning device, according to claim 1, further comprising protection means to cause the disconnection of the supply of the power source to the second body (6, 7, 9).

13. Room air-conditioning device, according to claim 12, wherein the protection means comprises a thermostat adapted to cut-off the source of electricity when the conductor wire (6) has reached a temperature greater than a predetermined threshold value.

14. Room air-conditioning device, according to claim 1, **characterized in that** the power source comprises a DC source of electricity.

15. Room air-conditioning device, according to claim 14, wherein the power source comprises at least one DC solar photovoltaic generator.

## Patentansprüche

1. Raumklimaanlage, umfassend:
- einen Rahmen (1, 2),
- einen ersten Körper (10) zum Emittieren negativer Ionen, welcher innerhalb des Rahmens (1, 2) angeordnet ist, um eine Ionisierung und Ausfällung der Teilchen, die in Suspension in der Atmosphäre des Raums vorliegen, zum Reinigen der Luft und zum Beseitigen des schlechten Geruchs zu veranlassen,
- Emissionsmittel (6, 7, 8, 9) zum Emittieren von Infrarotstrahlung, um die Wände und/oder Gegenstände, welche in dem Raum enthalten sind, zu trocknen, wobei die Emissionsmittel Folgendes umfassen:
- einen Infrarotstrahlung emittierenden zweiten Körper (7, 8, 9), welcher gestaltet ist, um Infrarotlicht durch Erwärmen zu emittieren, und
- eine Stromquelle zum Bereitstellen von Energie, um den zweiten Körper (7, 8, 9) zu erwärmen, wobei die Stromquelle eine Elektrizitätsquelle umfasst, welche mit dem zweiten Körper (7, 8, 9) durch einen elektrischen Leitungsdraht (6) verbunden ist,
wobei der zweite Körper (7, 8, 9) Folgendes umfasst:
- eine erste Platte (7) mit einer im Wesentlichen gleichmäßigen Dicke und **dadurch gekennzeichnet, dass** der zweite Körper ebenfalls
- eine zweite Platte (8) umfasst, welche auf einer ihrer Flächen mit einem gewundenen Kanal (9) ausgestattet ist, um den Leitungsdraht (6) aufzunehmen,
wobei der Leitungsdraht (6) zwischen der ersten Platte (7) und der zweiten Platte (8) eingeschlossen ist.

2. Raumklimaanlage nach Anspruch 1, wobei der erste Körper (10) ein Körper ist, welcher fähig ist, negative Ionen ohne Energieverbrauch zu emittieren.

3. Raumklimaanlage nach einem der Ansprüche 1 und 2, wobei der zweite Körper (7, 8, 9) die Form einer Platte aufweist.

4. Raumklimaanlage nach einem der Ansprüche 2 und 3, wobei der erste Körper (10) eine Keramikbeschichtung ist, welche negative Ionen emittiert.

5. Raumklimaanlage nach Anspruch 4, wobei die Keramikbeschichtung ein Anstrich ist, welcher silberdotierte Zeolithelemente umfasst.

6. Raumklimaanlage nach einem der Ansprüche 1 bis 5, wobei der zweite Körper (7, 8, 9) aus einem wärmeleitenden Material hergestellt ist.

7. Raumklimaanlage nach Anspruch 6, wobei das Material anodisiertes Aluminium ist.

8. Raumklimaanlage nach Anspruch 1, wobei sie ferner eine aseptische Verbindung enthält, um Bakterien und Keime aus der Atmosphäre zu beseitigen.

9. Raumklimaanlage nach Anspruch 8, wobei die aseptische Verbindung Nanosilber enthält.

10. Raumklimaanlage nach Anspruch 5 und 9, wobei der keramische Anstrich mit silberdotierten Zeolithelementen die auf Nanosilber basierte aseptische Verbindung ist.

11. Raumklimaanlage nach Anspruch 1, wobei die erste Aluminiumplatte (7) den keramischen Anstrich enthält.

12. Raumklimaanlage nach Anspruch 1, ferner Schutzmittel zum Veranlassen der Trennung der Versorgung des zweiten Körpers (6, 7, 9) aus der Stromquelle umfassend.

13. Raumklimaanlage nach Anspruch 12, wobei das Schutzmittel einen Temperaturregler umfasst, welcher angepasst ist, um die Elektrizitätsquelle abzuschalten, wenn der Leitungsdraht (6) eine Temperatur, die höher als ein vorbestimmter Schwellenwert ist, erreicht hat.

14. Raumklimaanlage nach Anspruch 1, **dadurch gekennzeichnet, dass** die Stromquelle eine Gleichstrom-Elektrizitätsquelle umfasst.

15. Raumklimaanlage nach Anspruch 14, wobei die Stromquelle mindestens einen fotovoltaischen Gleichstrom-Solargenerator umfasst.

## Revendications

1. Dispositif de climatisation d'une pièce comprenant :
- une armature (1, 2),
- un premier corps (10) pour émettre des ions négatifs, disposés à l'intérieur de l'armature (1, 2), pour générer une ionisation et une précipitation de particules présentes en suspension dans l'atmosphère de ladite pièce, pour purifier l'air et éliminer les mauvaises odeurs,
- des moyens d'émission (6, 7, 8, 9 pour émettre un rayonnement infrarouge, pour sécher les parois et/ou objets contenus dans la pièce, les moyens d'émission comprenant :
- un deuxième corps émettant un rayonnement infrarouge (7, 8, 9) configuré de sorte à émettre une lumière infrarouge en chauffant, et
- une source d'alimentation pour fournir de l'énergie pour chauffer le deuxième corps (7, 8, 9), la source d'alimentation comprenant une source d'électricité reliée au deuxième corps (7, 8, 9) par le biais d'un câble conducteur électrique (6),
dans lequel le deuxième corps (7, 8, 9) comprend :
- une première plaque (7) ayant une épaisseur sensiblement uniforme, et **caractérisé en ce que** le deuxième corps comprend également
- une deuxième plaque (8) équipée d'un canal d'enroulement (9) sur l'une de ses surfaces pour loger le câble conducteur (6),
dans lequel le câble conducteur (6) este renfermé entre la première plaque (7) et la deuxième plaque (8).

2. Dispositif de climatisation d'une pièce, selon la revendication 1, dans lequel le premier corps (10) est un corps pouvant émettre passivement des ions négatifs, sans consommer d'énergie.

3. Dispositif de climatisation d'une pièce, selon l'une quelconque des revendications 1 et 2, dans lequel le deuxième corps (7, 8, 9) a la forme d'une plaque.

4. Dispositif de climatisation d'une pièce, selon l'une quelconque des revendications 2 et 3, dans lequel le premier corps (10) est un revêtement en céramique émettant des ions négatifs.

5. Dispositif de climatisation d'une pièce, selon la revendication 4, dans lequel le revêtement céramique est une peinture comprenant des éléments en zéolithe dopée à l'argent.

6. Dispositif de climatisation d'une pièce, selon l'une quelconque des revendications 1 à 5, dans lequel le deuxième corps (7, 8, 9) est fabriqué en un matériau conducteur thermique.

7. Dispositif de climatisation d'une pièce, selon la revendication 6, dans lequel le matériau est de l'aluminium anodisé.

8. Dispositif de climatisation d'une pièce, selon la revendication 1, dans lequel il incorpore en outre un composé aseptique pour éliminer les bactéries et les microbes de l'atmosphère.

9. Dispositif de climatisation d'une pièce, selon la revendication 8, dans lequel le composé aseptique contient du nano-argent.

10. Dispositif de climatisation d'une pièce, selon les revendications 5 et 9, dans lequel la peinture en céramique avec des éléments en zéolithe dopée à l'argent constitue le composé aseptique à base de nano-argent.

11. Dispositif de climatisation d'une pièce, selon la revendication 1 dans lequel la première plaque en aluminium (7) incorpore la peinture en céramique.

12. Dispositif de climatisation d'une pièce, selon la revendication 1, comprenant en outre des moyens de protection pour générer la déconnexion de l'approvisionnement en source d'alimentation au deuxième corps (6, 7, 9).

13. Dispositif de climatisation d'une pièce, selon la revendication 12, dans lequel les moyens de protection comprennent un thermostat adapté pour couper la source d'électricité lorsque le câble conducteur (6) a atteint une température supérieure à une valeur seuil prédéterminée.

14. Dispositif de climatisation d'une pièce, selon la revendication 1, **caractérisé en ce que** la source en alimentation comprend une source CC d'électricité.

15. Dispositif de climatisation d'une pièce, selon la revendication 14, dans lequel la source en alimentation comprend au moins un générateur photovoltaïque solaire CC.
